Europäisches Patentamt

European Patent Office

Office européen des brevets

(11) Publication number: **0 405 592 A2**

# EUROPEAN PATENT APPLICATION

(21) Application number: 90112479.2

(22) Date of filing: 29.06.90

(51) Int. Cl.5: **C12Q 1/68**

(30) Priority: 30.06.89 JP 170685/89
30.06.89 JP 170686/89
26.07.89 JP 193130/89
26.07.89 JP 193131/89
30.08.89 JP 223773/89
30.08.89 JP 223774/89

(43) Date of publication of application:
02.01.91 Bulletin 91/01

(84) Designated Contracting States:
DE ES FR IT

(71) Applicant: SANYO CHEMICAL INDUSTRIES
LTD.
11-1, Ichinohashinomoto-cho
Higashiyama-ku, Kyoto-shi
Kyoto 605(JP)

(72) Inventor: Kawakatsu, Kunio, c/o Sanyo
Chemical Ind., Ltd.
1106-3, Minamisakura, Yasu-cho, Yasu-gun,
Shiga-ken, 520-23(JP)

(74) Representative: Kador & Partnerner
Corneliusstrasse 15
D-8000 München 5(DE)

(54) A method for detection of nucleic acid and reagents for their detection.

(57) The present invention relates to detecting methord for a specific nucleic acid through hybridization from single staranded nucleic acid and single standord nucleic acid acting complementarily, by using a labelled material having binding capacity to the hybridized double stranded nucleic acid under separation single stranded nucleic acid which was not subjected to hybridization.

The present invention possible to specifically detect the nucleic acid found in trance within serum or other fluids as well as the nucleic acid originated in biological contaminations within foods, time required for detection being substantially reduced thoreby. The invention is generally applicable to laboratory tests in clinic , food fabrication industries and so on.

FIG. 1

# A METHOD FOR THE DETECTION OF NUCLEIC ACID AND REAGENTS TO BE USED IN DETECTION THEREOF

Field of the invention

This invention relates to a method for the detection of nucleic acid and reagents to be used for this purpose.

Background of the invention

With the recent development of bio-technology, there occurs an ever growing need to detect these nucleic acids which show a specific nucleotide sequence. For this purpose following process is commonly known: the nucleic acid is first segmented using restriction enzyme, separated through electro-phoresis using gel of agar-agar or polyacrylamide and then physically or electrically transferred onto an absorption membrane made of nitro-cellulose or the like. The resultant material is transformed into a single stranded one which is then allowed to be hybridized with the single stranded nucleic acid labelled with an isotope for the subsequent detection through auto radiography. As an alternative is known an immunological detection process using an antibody against nucleic acid. Another prior art is desclosed in the Japanese Patent laid Open Publication No. 60-262055 (which corresponds to EP-A-0163220) and so on.

The conventional methods, however, have invariably a shortcoming which is related to the following complicated and time consuming procedures, e.g. adsorption of sample nucleic acid onto the membrane, preparation of gel, electrophoresis, transference to membrane, and auto radiography. In addition, the conventional ones are not suited for automatization, inadequately assured in security and inferior in term of specificity because nucleic acid have common structure to each kind of biological species.

In other words, where a single stranded nucleic acid is labelled with an isotope such as 32p as a label material upon immobilizing the subject nucleic acid under the conventinal process. higher sensitivity may be realized for detection, while at the same time it is nothing but a time-consuming process and also found inferior in security. In the detecting method using an antibody, an accuracy of detection is poor, because a nucleic acid has a poor property as immunogen and therefore it is difficult to provide an antibody adapted to a specific hybridized nucleic acid.

A prior art process employing an antibody is disclosed in the Japanese Patent Laid Open publication No. 60-262055 (which corresponds to EP-A-0163220). In comparison with the present invention, however, it does show a fatal disadvantage, that a RNA is essentially contained in one of nucleic acid made available for detection, in which case where the specific nucleic acid corresponds to RNA, an enhancement of nucleic acid in vitro for a higher sensitivity requires a complicated and time consuming procedure. Where a single stranded nucleic acid complementary to a specific nucleic acid corresponds to RNA, an advantageous procedure such as chemical synchesis for furnishing a lot of them as reagents is not made available.

As a matter of fact RNA shows an unusually higher sensitivity for pH-value and intermixed digestive enzyme and hence requires a technique, of which level is inevitably as high as is unable to be controlled by any clinical technologist with an averaged skill. RNA is therefore not suitable for the purpose of detection. In addition, RNA, when bio-synthesized from DNA, occasionally causes elimination or variation depending on the range of nucleic acid, which would pose a problem where detection will be intended for heredopathia. For this reason RNA could not be qualified as a clinical reagent, which accuracy, convenience and rapidness is required.

If a plurality of single stranded nucleic acids are made available, it is very advantageous because that vira and bacteria with mutant may be detected using one and same reagent and that a plurality of disorders may be searched at a time. However, vira and becteria being externally influenced are easy to develop mutant, so that diagnosis and therapeutic measure often faces a difficulty. Papilloma virus or influenzavirus, for example, have many different kinds of mutant, which would appear as a varied one whenever the lattor will become prevalent especially. In view of the foregoing, such reagent which is limited in application only to a kind of nucleic acid under test will pose a problem that could mislead the diagnosis in question.

Summary of the invention

The present invention is capable to eliminate the aforementioned disadvantages of the conventional processes and to provide a detective process and reagents for the same which are particularly suitable for mass-screening. The objective of the invention is to provide a method and reagents useful for the same which make it possible to specifically detect the nucleic acid under test contained in trace within serum and other body fluids or of bio-infectious origin in foods and also to substantially reduce time expenditure required for the implementation of process.

The effort of the inventors was in effect devoted to the research for development of a method which makes it possible to detect the hybridized nucleic acid in a remarkably rapid, secured and selective manner and is readily to be automated as well as reagents useful for the same and finally the present invention has come successfully realized.

In order to accomplish the above objects, this invention includes as followes:

(1) A method for detecting a specific DNA through a labelled double stranded DNA comprising:

(a) attaching single stranded DNA fragments to a carrier,

(b) hybridizing at least a portion of the single stranded DNA fragments with complementary single stranded DNA fragments to form double stranded DNA, and

(c) attaching a labelled material with labelling compound to the double stranded DNA fragments.

(2) Reagents adapted for the method in accordance with above mentioned (1) to be used for the said double stranded nucleic acid (DNA* -DNA hybrid) comprising single stranded DNA * and material with binding capacity labelled with a labelling compound being reactive with double tranded nucleic acid (DNA* -DNA hybrid) obtained from hybridization with single stranded DNA* .

(3) A method for detecting a specific nucleic acid through a labelled double stranded nucleic acid comprising:

(a) hybridizing at least a portion of a single stranded nucleic acid fragments with complementary single stranded nucleic acid fragments to form double stranded nucleic acid,

(b) decomposing the single stranded nucleic acids fragments which are not hybridized, and

(c) attaching a labelled material with labelling compound to the double stranded nucleic acid fragments.

(4) Reagents adapted for the method under above mentioned (3) to be used for detecting double stranded nucleic acid comprising: material(a) for decomposing a single stranded nucleic acid which was not hybridized and material (b) with binding capacity labelled by a labelling compound, which is capable to react with a nucleic acid which was made to double strands through hybridization.

Brief description of the drawings

This invention will now be described in details with reference to the following drawings, in which:

Fig.1 is a diagram illustrating the results from the detection of nucleic acid set forth in the Example 1 and Comparative Example 1;

Fig.2 is a diagram illustrating the results from the detection of nucleic acid set forth in the Example 3 and Comparative Example 2;

Fig.3 is a diagram illustrating the results from the detection of nucleic acid set forth in the Example 5 and Comparative Example 3;

Fig.4 is a diagram illustrating the results from the detection of nucleic acid set forth in the Example 7 and Comparative Example·4;

Fig.5 is a diagram illustrating the results from the detection of nucleic acid set forth in the Example 9 and Comparative Example 5; and

Fig.6 is a deagram illustrating the results from the detection of nucleic acid set forth in the Example 11 and Comparative Example 6.

Detailed description of the invention

Referring to the present invention, the term "nucleic acid" under the description means either of single- or double stranded nucleic acid, which shall, however, be so specified each time, when appropriate. It also comprises every and all nucleic acids with more than 1 of bases, irrespective of base number.

It also comprises not only those, in which the contained nucleotide is of natural origin, but also those, in which the nucleotide is artificially modified.

In addition, the term "single stranded nucleic acid" shall be interpreted literally but it is understood that it contains also such nucleic acid which evolved as the result of hybridization made between single stranded nucleic acids with different lengths of strand.

The single stranded nucleic acid under the present invention also means such a nucleic acid, of which nucletide sequence is found complementary to the counterpart of specific ones under detection test. Above mentioned definitions are also applied to the "DNA" which is one kind of nucleic acid.

As for deoxyribo nucleic acid (DNA), it may be prepared in such a manner that a double stranded nucleic acid of natural origin is devided into single stranded nucleic acids followed by refinement or alternatively it may be synthesized artificially. The latter case involves certain chemical procedures such as a process, through which nucleotides of following 4 kinds are successively bonded: adenine, guanine, cytosine and thymine: and a synthetic process using enzyme.

Said chemical procedures would be preferred due to higher purity and mass production capability available thereby.

In case of ribo nucleic acid (RNA) any single stranded nucleic acid may be made available without further processing.

Referring to the base number single stranded nucleic acid, it usually counts to 1 to 10000. In general, larger count is more preferable from accuracy and sensitivity of detection.

However, where the count of 500 is exceeded, remarkable variation would no longer become evident. The most desirous count is 5 to 100, in which case a complementary strand may be identified easily and the required accuracy and sensitivity maintained adequately.

Also single stranded nucleic acid may be used being immobilized on a carrier. As the carrier, siliceus inorganic carrier, for examples, glass (porous glass, ground glass), silica-gel, bentonite, etc., magnetic carrier and organic carrier (plastics such as nitro-cellulose, cellulose acetate, Nylon, polyethylene, polyropylene, polystylene, polyvinylchloride, polyvinylidenfluoride, silicon, polyurethan, dextran, filter paper, etc.) may be made available. Glass, nitro-cellulose, cellulose acetate polyvinylidenfluoride, Nylon is more preferable on account of their binding capacity. Glass is the best choice, because of its reasonable price and easy-to-prepare characteristic. Where glass is made in use, the glass surface area, onto which single stranded nucleic acid is to be fixed, may be advantageously roughened through sand blast, etc. for the purpose of enhancement of the same.

To immobilize single stranded nucleic acid, physical adsorption and chemical bonding is most useful. In case of physical adsorption, the solution containing single stranded nucleic acid to be adsorbed (e.g. phosphoric acid buffer solution containing nucleic acid of 10μg/ml. etc.) is conveniently made to come into contact with the carrier. The carrier may be heated up, for example, 2 hours at 80 °C so that the binding would become more rigid. Carriers carrying functional group such as amino- or carboxyl group and single stranded nucleic acid may be bonded together through bifunctional crosslinking agent (e.g. glutaraldehyde etc.) or bi-functional activator (e.g. water-soluble carbodiimide, carbodiimidazol,N-hydroxy succinimide, bis(sulfosuccinimidyl) suberate,disulfosuccinimidyl tartarate, etc.) so as to achieve a covalent bond. Of the above two processes, chemical bonding is considere a better choice, since it can afford an enhancement of binding strength and binding stability of single stranded nucleic acid and an improvement of reactivity in hybridization.

In connection with covalent bond, certain distance between carrier surface and binding site (end base) of single stranded nucleic acid may be advantageously maintained, in order that the reactivity of hybridization will be improved, which in-turn will enhance the sensitivity. For this purpose, where carrier is bonded with single stranded nucleic acid through crosslinking agent or activator, certain compound is preferably incorporated, so that functional group may have a molecular length exceeding certain limit (e.g. 1nm) either on the surface of carrier or on the binding site of single stranded nucleic acid.

In addition, the single stranded nucleic acid discussed here may be either of single sort for the determination of single object or of more the 2 sorts (e.g. 2 to 30 sorts) for the determintion of a plurality of objects. For those diseases which are attributable to exo-pathogenic substances such as bacteria, virus, reckettsiae, protozoans and spirochete, etc. and / or those genetic disorders which are attributable to mutation of nucleotide sequence of nucleic acid, i.e. substitution, insertion, deletion, rearrangement of base, it is possible to select several sorts of objects within the single stranded nucleic acid specific to these diseases. Where several different nucleotide sequences may exist even in the same kind of discase, mutant of bacteria and virus or said genetic disorders may be the objects under search. In practice, the pathogeneic substances cover but not limited to the followings: bacteria such as Shigella, Corynebacterium diphtheriae, Clostridium botulinum, Salmonella; virus such as Adenoviridae Human adenovirus, Orthomyxoviridae Influanza virus, Papovaviridae Human papilloma virus. Genetic disorders involve but not limited to the followings: familial hypercholesteremia, Type III hyper lipemia, Thalassemia, Hemophilia, Diabetes mellitus, cancer and so on. Referring now the single stranded nucleic acid acting complementarily within specimen, microorganism, virus, fishes, fowls, plant, animal, particularly mammal (inclusive mankind) are counted in the origin of such nucleic acid being discussed in connection with double stranded nucleic acid of natural origin. Virus may be roughly divided into following two categories: one comprising single stranded nucleic acid and the other double stranded nucleic acid. Both may either coexist or exist separately.

As different kinds of nucleic acid may be contained in the specimen under test, those nucleic acid which do not act complementarily may be contained therein in addition to those acting complementarily with single stranded nucleic acid.

However, there is eventually found such case where no nucleic acid will exist other than complementary ones.

The nucleic acid discussed here is generally extracted from cells, and subjected to a fragmentation through such chemical means as restriction enzyme (e.g. EcoR$_I$, Hind$_{III}$, Pst$_I$, Bgl$_I$, etc.) or through physical means such as supersonic wave, etc. For the purpose of routine preparation through restriction enzyme, 100mM-Tris buffer solution (pH 7.5) containing 0 to 1.0 M-NaCl is added with 0.1 to 100 $\mu$g of nucleic acid specimen and 0.1 to 100 units of restriction enzyme, and then shaked for 10 to 120 min. under 20 to 45°C The said procedure is disclosed in detail in Tom Maniatis: Molecular Cloning, Cold Spring harbor laboratory, p104, 1982. In the event where nucleic acid of natural origin is found short in quantity, then such a method may be found of use, in which nucleic acid is allowed to proliferate enough to be adequately detected within test tubes using some kinds of polymerase.

For the purpose preparation of complementary single stranded nucleic acid, segmented nucleic acid may be heated up or denaturated using alkaline solution. The method in practice comprises but not limited to the following steps: segmented nucleic acid is heated for approximately 1 to 30 min. under the temperature of approximately 60 to 150°C, preferably for approximately 10 to 20 min. under approximately 80 to 100°C, whereas, when degenerated, it is added with an appropriate quantity of sodium hydroxide solution in water, allowed to stand for approximately 10 to 40 min. under room temperature and then neutralized.

For the purpose of hybridization of nucleic acid, the specimen discussed in the above is intermixed with single stranded nucleic acid, and shaked for approximately 10 min. to 10hrs. under the temperature of around 25 to 80°C the said methods may be made easier to be carried out when reagents kit (Rapid hybridization system "Multi-Prime" Amersham Japan Co., Ltd.) is made available (it is available in market).

Binding substances being reactive with those nucleic acids which became double stranded through hybridization involve such substances which may be selectively captured into molecules of hybrid, particulary between strands which have been bonded together through hydrogen bonding. e.g. such reagents as antibody, double stranded nucleic acid-binding protein, etc. Of those protein, particularly antibody is the best choice in view point of security and stability of resultant compounds. Since the surplus nuclei acid remaing left in the system is not subjected to decomposition but caused to directly react with antibody, the antibody discussed here must be characterized with a higher specificity to the resultant hybrid. In connection with this, both poly- and monoclonal antibodies are suitable for the purpose intended, but the latter is preferred.

Monoclonal antibody may be produced through a routine method in such a way that fused cells-producing monoclonal antibody is first produced; immunized animals (e.g. mice, rats, rabbits, etc.) are made immuzed through ip(intraperitoneal) infusion of nucleic acid of either natural- or artificial origin so as to cause spleen cells or lymphcyte to be produced about one or 2 months later; the latter so produced is then made intermixed with myeloma cell in the presence of fusion accelerator such as PEG-4000 etc. and allowed to stand for several min. under room temperature; out of these cells only fused cells are selectively incubated on HAT medium, so that only the selectively incubated cells is made cloned through critical dilution method so as to ·acquire fused cells producing monoclonal antibody; the latter cells is incubated, so that the desired monoclonal antibody may be obtained from supernatant through routine procedure, or alternatively from ascites which was sampled from ascites tumor which was caused to develop through ip infusion of fused cells to mouse, either of the above procedure being followed by a rectification. The rectification may readily take place using a ready-equipped column available in market. This method is disclosed in detail in "Zoku Seikagaku Jikkenkoza 5 - Men-eki Seikagaku Kenkyuho" compiled by Japan Biological Society, Page 1~84, Tokyo Kagaku Dohjin.

Referring now to another detection method of the present invention. The same terminology has the same meaning both in the aforsaid detection method and the another detection method of the present inventions.

For the purpose of eliminating those nucleic acid which failed to achieve hybridization, a method incorporating digestion and decomposition through enzyme is made available, since selective eliminating is needed for the same. Suitable for the above purpose are those enzymes which specifically act on single stranded nucleic acid, e.g. enzymes segmenting single stranded nucleic acids, such as S1-Nuclease, Mung Bean Nuclease, P-1-Nuclease, Exonuclease $_{-I,-III}$, and$_{-VII}$, Deoxyribonuclease-$_I$, Ribonuclease-H. etc. Of these S1-Nuclease, Mung Bean Nuclease and Bal-31-Nuclease are preferred in specificity. With these enzymes decomposition may readily take place in such a manner that specimen containing hybridized nucleic acid of approx. 1pg to 1$\mu$g is added with single stranded segmenting enzyme of 0.001 to 1000 units: and then shaked for 5 to 120 min. under 20 to 50°C. Practical method is discloed in detail Tom Maniatis; Molecular Cloning. Cold Spring Harbor Laboratory P.237, 1982.

Suitable as the binding substance being reactive with hybridized nucleic acid are those substances which may be caught into the molecules of hybrid, particularly inbetween the strands bonded together through hydrogen bond, e.g. chemical reagents such as Ethidium bromide, Propidium diiodie, etc. or proteins such as antibody, double stranded nucleic acid binding protein. etc.

Of these proteins, particularly antibodies are preferred in view point of security for material and stability of resulatant substance. The antibody is not needed to act on hybrid specifically, because those nucleic acid which were found unable to be hybridized in practice will be eliminated out of system through decomposing process. The antibody may be either poly-clonal or mono-clonal. These antibodies may be produced through routine method.

Labelling compounds to label antibody are isotopes, fluorescent chromophores, luminescent chromophros, enzymes and compounds capable to indirectly bond the same. In practice they comprise but not limited to the following substances: isotopes of $[^{32}P],[^{35}S],[\,^3H],[^{14}C]$, etc, as labelling materials: fluorescein isothiocyanate (FITC), tetramethylrhodamine isothiocyanate (RITC), Acridine orange,fluorescein, etc. as fluroescent chromophore: luminol and luciferin, etc. as luminescent chromophore; peroxidase, alkaline phosphatase, $\beta$-galactosidase, glucose oxidase, etc. as enzyme; and biotin (capable to be bouded by avidin), antibody (capable to be bonded by antigen and second antibody), etc. as indirectly bondable compounds.

For the purpose of labelling the process set forth in "Zoku Seikagaku Jikkenkoza 5, Meneki Seikagaku Kenkyuho" compiled by Japan Biological Society, p102 to 112, Tokyo Kagaku Dojin, may be readily made available.

To detect through binding substance to nucleic acid, those procedures which are similar to the routine immunological measurement (enzyme immunoassay or radio-immunoassay) are of use. Of these, those procedure using antibody which was provisionally labelled for the nucleic acid to be hybridized or those using secondary labelled antibody against such antibody which was made bonded to nucleic acid are of interest.

Referring to the present invention, the term "detection" under the description means include determination.

Reagents using detection of nucleic acid under the present invention may contain certain auxiliary components for making the reagent more easy-to-use, in addition to the essential components. For instance solvent to solve a solid matter, cleaning agent to make it possible to eliminate component which became unnecessary in the course of reacting process, reaction suspending agent to suspend the on-going reaction of enzyme and substrate used for measuring of enzymatic activity, etc. or any combination thereof belong to said category of components.

Those procedures and reagents proposed in the present invention provides the possibility to realize a higher specificity and a more rapid and secured measurement than is in the case of conventional ones, i.e. they are capable to specifically detect such nucleic acid with a higher sensitivity and accuracy which is contained in trace within serum or other fluids or of bio-infectious origin contained in foods as well as to reduce the time expenditure required for the same. The invention is considered specifically suitable for the application in the fields of clinical laboratory and food processing industry.

Embodiments

The examples and comparative examples of the present invention placed hereunder: it is understood, however, that the invention shall not be considered solely limited therto.

Example 1 (Detection of specific nucleic acid)

1) Preparation of single stranded nucleic acid complementarily acting within the specimen

As a DNA, pBR322(produced by Takara-Shuzo Co., Ltd.) and restriction enzyme BglI(produced by TOYOBO CO., LTD. through dividing pBR322 into 3 segments), both are available in marketplace, were completely digested to the reactive conditions recommended by the relevant manufacturer followed by routine phenol-extraction ethanol-precipitation, and refining. The resultant material was further subjected to refining and then subjected to thermal treatment at 100°C for 10min., the concentration being adjusted to 100 µg/ml, which was made in use for a series of tests described hereunder as the nucleic acid reagent (A).

2) Preparation of immobilized single stranded nucleic acid

As the single stranded nucleic acid, pBR322 PrimerH(produced by Takara-Shuzo Co., Ltd. with nucleotide sequence complementary to pBR322; base number = 16) available in market was made in use. 300 µl of PrimerH solved in 0.1M carbon acid buffer solution (pH = 9.8) to a concentration of 0.1µg/ml was upon intermixed with glass beads, shaked for 2hrs. under room temperature. It was then washed twice using 500µl of 0.02M phosphoric acid buffer solution (pH = 7.5) containing 0.01wt% (% by weight) Tween (0.01wt% Tween-PBS surfactant), added with 1wt% BSA solved in PBS and finally shaked for 10min. under 37 °C. The material so resulted was made in use for a series of tests described hereunder as the immobilized single stranded nucleic acid reagent (B).

3) Preparation of anti nucleic acid monoclonal antibody

A monoclonal antibody to a nucleic acid was prepared using an improved procedure, of which orignal is referenced in the literature [Oskar S. Frankfurt:Experimental Cell Research) vol. 170, p369-380, 1987].

In short, calf thymus DNA (type I, produced by Sigma Chemical Co.) was digested using S1-nuclease (produced by Takara-Shuzo Co., Ltd.) and then bonded to methylated bovine serum albumin so as to be made in use as an immune substance. 200µg of the latter together with a complete adjuvant of Freund was ip(intraperitoneal) infused into a BALB/C mouse for the purpose of immunization thereof. Immune substance in the same quantity as above was each time ip infused 3-, 5-, 7 weeks after the initial infusion. Further the same quantity of immune substance was used for the purpose of boosting 2 weeks later. The spleen was extirpated 3 days later to sample spleen cells. Complete cell sample of the spleen, upon completion of washing by RPMI medium, was admixtured with 2 ×10⁷ celles of mouse myeloma (33-NS1/1-Ag 4.1) so as to be fused together for 1 min. within 1 ml of RPMI 1640 medium containing 42.5wt% of polyethylene glycol having 1540 of average molecular weight and 7.5wt% of dimethylsulfoxide both of 37 °C. The resulted fused cell was washed and made suspended under HAT medium (RPMI 1640 medium containing hypoxanthin, aminopterin, thymidim and 10wt% of fetal calf serum (FCS) and then transferred onto a plurality of microplates for 2 weeks' incubation. Of the proliferated cells 2 cells showing a desired antibody activity in the order of IgG were obtained through relevant survey of antibody activity of the proliferated cells. Each of the said 2 cells was ip infused into a mouse to sample ascites thereof. The latter was rectified and isolated using AFFI-GEL(Trade name), Protein A MAPS kit (produced by Biorad Co.) into antibodies Nos.1 and -2 to be used for a series of test described hereunder.

4) Preparation of a labelled antibody

No.1 of antibody mentioned above was bound to peroxidase(POD) through a process set forth in [S.Yoshitake M. Imagawa, E. Ishikawa et al.: J. Biochem., vol.92(1982) p.1413~1424] to obtain a labelled antibody. The latter was then diluted with a buffer solution containing 1wt% bovine serum albumin(BSA) down to 10µg/ml to be made in use as a reagent containing labelled antibody(C).

5) Hybridization

PBS's of 500µl per each containing 0-, 5-, 10- and 20µl of (A), each of which was preheated for 5 min. under 95 °C and then immediately cooled on ice were intermixed with (B) to be brought to a hybridization through shaking for 2hrs. under 60 °C. Glass beads thereupon were washed twice with 0.01wt% Tween-PBS(500µl).

6) Detection

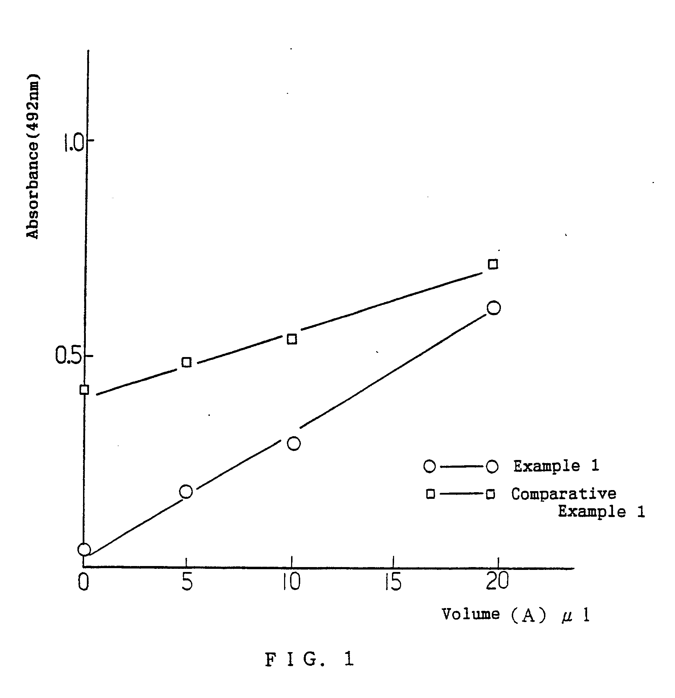

The reactants obtained under 5) added with 100µl of (C) and 400 µl of PBS were shaked for 60 min. under 37 °C and then washed with physiological salt solution. The mixtures were added with 500 µl of substrate solution (orthophenylendiamine solution containing hydrogen peroxide) and then shaked for 30 min. under 37 °C. The reaction under progress was suspended using 3ml of 1.5N sulfuric acid. The mixtures were subjected to an absorbance measurement in term of specific wave length of 492nm to determine activity of enzyme being bonded to the glass beads. The results from the above detections of a nucleic acid are illustrated in Fig.1.

Comparative Example 1

The Comparative Example 1 was subjected to detection of nucleic acid through the same procedure as is the case of Example 1 except that anti nucleic acid antibody available in marketplace (Chemicon International Co.) was made in use. The results from the above detections of the Comparative Example 1 are illustrated in Fig.1. As seen from the Fig.1 it becomes evident that only such nucleic acid which was subjected to hybridization may be detected specifically, securely and rapidly by the method of the present invention.

Example 2

Those detective reagents under the present invention were prepared such that the following reagents were individually filled in a plug sealed flask.

1) Nucleic acid reagent (A) 1.2ml
2) Fixed single straned nucleic acid reagent (B) 100pcs.
3) Labelled antibody reagent (C) 12ml
4) PBS 120ml
5) 0.01wt% Tween-PBS 120ml
6) Physiological salt solution 1000ml
7) Substrate solution 60ml
8) Sulfric acid (1.5N) 350ml

These reagents were useful for the detection set forth in the Example 1. It is understood that the reagents 2) and 3) act as essential components for the present invention particularly in the above embodiment.

Example 3 (Detection of a plurality of specific nucleic acids)

1) Preparation of single stranded nucleic acid complementarily acting within the specimen

pBR322 DNA and M13mp19RFDNA (both produced by Takara-Shuzo Co., Ltd. and available in marketplace) were completely digested using restriction enzyme Hind ₁₁₁ (produced by Takara Shuzo Co., Ltd.) under the reactive conditions recommended by the manufacture to be rectified through routine phenol extraction, ethanol precipitation and others. The each resultant substance was subjected to a thermal treatment for 10min. under 100 °C.

Each specimen was then mixed together so as to be adjusted in concentration 100μg/ml per each. They are to be used for a series of tests described hereunder as the nucleic acid reagent (E).

2) Preparation of immobilized single stranded nucleic acid

As a single stranded nucleic acid pBR322 Primer H(produced by Takara-Shuzo Co., Ltd. having 16 of bases numbers and nucleotide sequence which act complementarily with pBR322) and M13p Primer M3(prodeuced by Takara-Shuzo Co., Ltd. having 17 of bases numbers and nucleotide sequence which act complementarily with M13mp 19RFDNA) were made available.

Each Primer solved in 0.1M carbon acid buffer solution (pH9.8) is mixed to a concentration of 0.1μg/ml, of which 300μl is intermixed with glass beads and then shaked for 24hrs. under room temperature. The mixture was washed twice using 500ml of 0.02M phosphoric acid buffer solution containing 0.01wt% Tween(pH7.5) (0.01wt% Tween-PBS), added with 1% BSA solved in PBS and then shaked for 10min. under 37 °C. The reagent so resulted is to be used for a series of tests described hereunder as the immobilized single stranded nucleic acid reagent (F).

And also, the immobilized single stranded nucleic acid reagent (B) under Example 1 was made in use here.

3) Preparation of anti nucleic acid monoclonal antibody

- under same procedure as was in the Example 1 -

4) Preparation of labelled antibody

- under same procedure as was in the Example 1 -

5) Hybridization

PBS's of 500μl per each containing 0-, 5-, 10- and 20μl of reagent (E) preheated for 5 min. under 95 °C then immediately cooled in ice, mixed with either (B) or (F) were brought to hybridization through shaking for 2hrs. under 60°C. The glass beads were washed twice using 0.01wt% Tween PBS (500μl).

6) Detection

- under same procedure as was in the Example 1 -

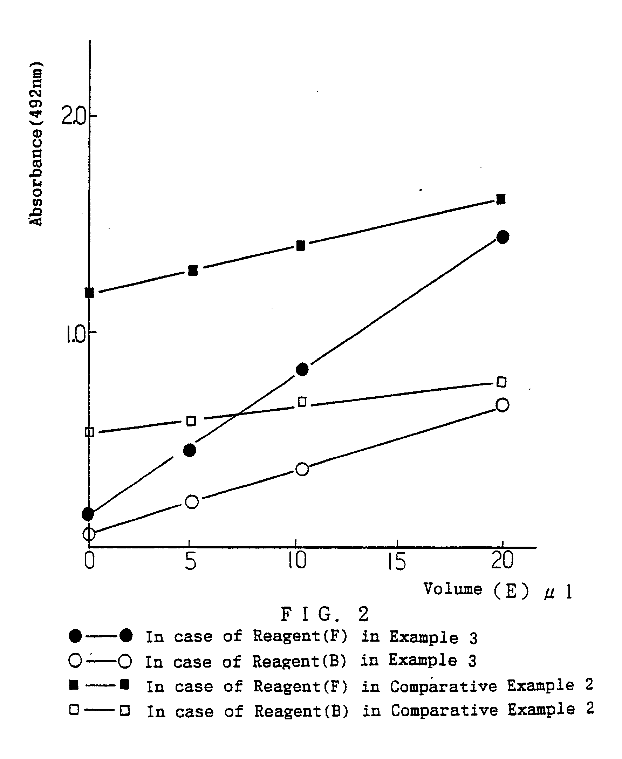

The results from the above detections of a nucleic acid are illustrated in Fig. 2.

Comparative Example 2

The Comparative Example 2 was subjected to detection of nucleic acid through the same procedure as is the case of Example 3 except that anti nucleic acid antibody available in marketplace (Chemicon International Co.) was made in use. The results from the above detections of the Comparative Example 2 are illustrated in Fig. 2. As seen from the Fig.2 it becomes evident that only such nucleic acid which was subjected to hybridization may be detected specifically, securely and rapidly by the method of the present invention.

Example 4

Those detective reagents under the present invention were prepared such that the following reagents were individually filled in a plug sealed flask.

1) Nucleic acid reagent (E) 2.4ml
2) Fixed single straned nucleic acid reagent (F) 100pcs.
3) Fixed single straned nucleic acid reagent (B) 100pcs.
4) Labelled antibody reagent (C) 24ml
5) PBS 240ml
6) 0.01wt% Tween-PBS 240ml
7) Physiological salt solution 2000ml
8) Substrate solution 120ml
9) Sulfric acid (1.5N) 700ml

These reagents were useful for the detection set forth in the Example 3. It is understood that the reagents 2), 3) and 4) act as essential components for the present invention particularly in the above embodiment.

Example 5 (Detection of specific nucleic acid)

1) Proparation of single stranded nucleic acid complementarily acting within the specimen

- under same procedure as was in the Example 1 -

2) Preparation of immobilized single stranded nucleic acid

As the single stranded nucleic acid oligonucleotide having the same nucleotide sequence as is in the pBR322 Primer H available in marketplace and having its 5'-end (OH-residue) aminoalkylated using carbodiimidazol and hexamethylendiamine was obtained.

As the carrier ground glass beads of 6mmØ with their surface amidated using γ-aminopropyl silane were made available.

The ground glass beads were intermixed with disuccinimidyl suberate (DSS) and the said aminoalkylated nucleotido to allow a reaction to proceed for 24hrs. under room temperature. The reactant so resulted had its concentration of nucleotide adjusted to 0.1 μg/ml and was subjected to the same treatment as was in the Example 1 to be made in use for a series of tests described hereunder as the fixed single stranded nucleic acid reagent (G).

3) Preparation of anti nucleic acid monoclonal antibody

- under same procedure as was in the Example 1 -

4) Preparation of labelled antibody

- under same procedure as was in the Example 1 -.

5) Hybridization

PBS's of 500μl per each containing 0-, 5-, 10- and 20μl of reagent (A) preheated for 5 min. under 95 °C then immediately cooled in ice, mixed with (G) was brought to hybridization through shaking for 2hrs. under 60 °C. The glass beads were washed twice using 0.01wt% Tween PBS (500μl).

6) Detection

The reactants obtained under 5) were added with 100μl of reagent (C) and 400μl of PBS, shaked for 6omin. under 37 °C and then washed using physiologic salt solution. They were then added with 500μl of substrate solution (orthophenylendiamine solution containing hydrogen peroxide) and shaked for 3omin. under 37 °C. The reaction under progross was suspended using 3ml of 1.5N sulfuric acid.

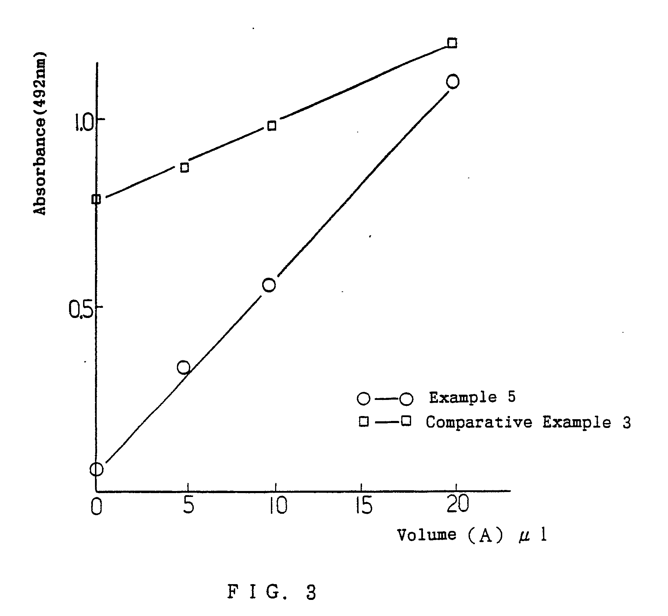

The absorbance of the solution was measured in term of the wave length of 492nm to determine the activity of enzyme bonded to glass beads. The results for the above detection of a nucleic acid are illutrated in Fig. 3.

Comparative Example 3

The Comparative Example 3 was subjected to detection of nucleic acid through the same procedure as is the case of Example 5 except that anti nucleic acid antibody available in marketplace (Chemicon International Co.) was made in use. The results from the above detections of the Comparative Example 3 are illustrated in Fig. 3.

It is evident that a constant molccule length maintained between the carrier and the single stranded nucleic acid advantageously serves for realizing a higher sensitivity and that only those nucleic acids which complementarily act with single stranded nucleic acid through enzyme treatment may be specifically, securely and rapidly detected.

Example 6

Those detective reagents under the present invention were prepared such that the following reagents were individually filled in a plug sealed flask.

1) Nucleic acid reagent (A) 1.2ml
2) Fixed single straned nucleic acid reagent (G) 100pcs.
3) Labelled antibody reagent (C) 12ml
4) PBS 120ml
5) 0.01wt% Tween-PBS 120ml
6) Physiological salt solution 1000ml
7) Substrate solution 60ml
8) Sulfric acid (1.5N) 350ml

These reagents were useful for the detection set forth in the Example 5. It is understood that the reagents 2) and 3) act as essential components for the present invention particularly in the above embodiment.

Example 7 (Detection of specific nucleic acid)

1) Preparation of single stranded nucleic acid complementarily acting within the specimen

As a DNA, pBR322(produced by Takara-Shuzo Co., Ltd.) and restriction enzyme BglI(produced by TOYOBO CO., LTD. through dividing pBR322 into 3 segments), both are available in marketplace, were completely digested to the reactive conditions recommended by the relevant manufacturer followed by routine phenol-extraction. ethanol-precipitation, and refining. The resultant material was further subjected to refining and then subjected to thermal treatment at 100°C for 10min. the concentration being adjusted to 100 $\mu$g/ml, which was made in use for a series of tests described hereunder as the nucleic acid reagent (A).

2) Preparation of immobilized single stranded nucleic acid

As the single stranded nucleic acid, pBR322 PrimerH(produced by Takara-Shuzo Co., Ltd. with nucleotide sequence complementary to pBR322; base number = 16) available in market was made in use.
100 $\mu$l of PrimerH solved in 0.1M carbon acid buffer solution (pH = 9.8) to a concentration of 0.1$\mu$g/ml was poured into polystyrene tube, shaked for 2hrs. under room temperature. It was then washed twice using 500$\mu$l of 0.02M phosphoric acid buffer solution (pH = 7.5) containg 0.01wt% (% by weight) Tween (0.01wt% Tween-PBS surfactant), added with 1wt% BSA solved in PBS and finally shaked for 10min. under 37°C. The material so resulted was made in use for a series of tests described hereunder as the immobilized single stranded nucleic acid reagent (B).

3) Preparation of a labelled antibody

Anti nucleic acid antibody available in marketplace (Chemicon International Co.) was bound to peroxidase(POD) through a process set forth in [S. Yoshitake, M. Imagawa, E. Ishikawa et al.: J. Biochem vol.92 (1982) p.1413~1424] to obtain a labelled antibody. The latter was then diluted with a buffer solution containing 1wt% bovine serum albumin (BSA) down to 10 $\mu$g/ml to be made in use as a reagent containing labelled antibody(C).

4) Hybridization

PBS's of 500$\mu$l per each containing 0-, 5-, 10- and 20$\mu$l of (A), each of which was preheated for 5 min. under 95°C and then immediately cooled on ice were intermixed with (B) to be brought to a hybridization through shaking for 2hrs. under 60°C and then washed twice with 0.01wt% Tween-PBS-(500$\mu$l).

5) Enzyme decomposition

S1-NUCLEASE (produced by Takara-Shuzo Co., Ltd.) available in marketplace having its concentration adjusted to 10 units/ml through a buffer solution recommended by the manufacturer was made in use as the reagent containing digestive enzyme (D). 500$\mu$l of the said buffer solution containing 10$\mu$l of (D) was added to the reactant under 4) allowing reaction to proceed for 10min. under 37°C and then washed twice using PBS containing 0.01wt% Tween (500 $\mu$l) as was the case in 4).

6) Detection

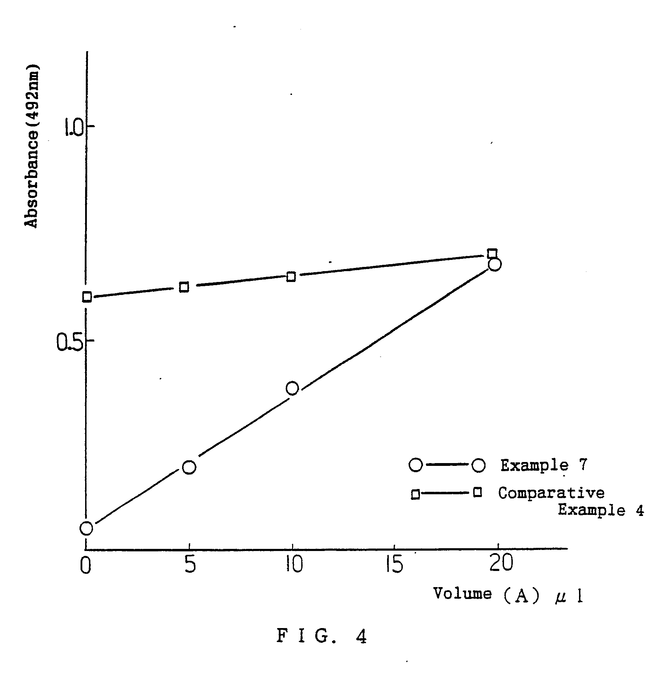

The reactants obtained under 5) added with 100$\mu$l of (C) and 400 $\mu$l of PBS were shaked for 60 min. under 37°C and then washed with physiological salt solution. The mixtures were added with 500 $\mu$l of substrate solution (orthophenylendiamine solution containing hydrogen peroxide) and then shaked for 30 min. under 37°C. The reaction under progress was suspended using 3ml of 1.5N sulfuric acid. The mixtures were subjected to an absorbance measurement in term of specific wave length of 492nm to determine activity of enzyme being immobilized to the glass beads. The results from the above detections of a nucleic acid are illustrated in Fig. 4.

Comparative Example 4

In case of Comparative Example 4, enzyme activity was determined in the same manner as in the Example 7 except that such nucleic acid which was not subjected to hybridization under the Example 7 was not digested using enzyme. The result obtained from the comparative Example 4 is illustrated in Fig. 4.

As a result it is evident that only such nucleic acid which acts complementarily with single stranded nucleic acid through enxyme digestion may be specifically, securely and rapidly detected.

Example 8

Those detective reagents under the present invention were prepared such that the following reagents were individually filled in a plug sealed flask.

1) Nucleic acid reagent (A) 1.2ml
2) Fixed single straned nucleic acid reagent (B) 100units
3) Labelled antibody reagent (C) 12ml
4) Digestive enzyme reagent (D) 1.2ml
5) Buffer solution for use digestive enzyme reagent 60ml
6) PBS 120ml
7) 0.01wt% Tween-PBS 240ml
8) Physiological salt solution 1000ml
9) Substrate solution 60ml
10) Sulfric acid (1.5N) 350ml

These reagents were useful for the detection set forth in the Example 7. It is understood that the reagents 2). 3) and 4) act as essential components for the present invention particularly in the above embodiment.

Example 9 (Detection of a plurality of specific nucleic acids)

1) Preparation of single stranded nucleic acid complementarily acting within the specimen

pBR322 DNA and M13mp19RFDNA (both produced by Takara-Shuzo Co., Ltd. and available in marketplace) were completely digested using restriction enzyme Hind ₍ₗₗₗ₎ (produced by Takara Shuzo Co., Ltd.) under the reactive conditions recommended by the manufacture to be rectified through routine phenol extraction, ethanol precipitation and others. The each resultant substance was subjected to a thermal treatment for 10min. under 100°C.

Each specimen was then mixed together so as to be adjusted in concentration 100 μg/ml per each. They are to be used for a series of tests described hereunder as the nucleic acid reagent (E).

2) Preparation of immobilized single stranded nucleic acid

As a single stranded nucleic acid pBR322 Primer H(produced by Takara-Shuzo Co., Ltd. having 16 of bases numbers and nucleotide sequence which act complementarily with pBR322) and M13p Primer M3(prodeuced by Takara-Shuzo.Co., Ltd. having 17 of bases numbers and nucleotide sequence which act complemontarily with M13mp 19RFDNA) were made available.

Each Primer solved in 0.1M carbon acid buffer solution (pH9.8) is mixed to a concentration of 0.1μg/ml, of which 100μl was poured into polystyrene tube and then shaked for 24hrs. under room temperature. The mixture was washed twice using 500ml of 0.02M phosphoric acid buffer solution containing 0.01wt% Tween(pH7.5) (0.01wt% Tween-PBS), added with 1% BSA solved in PBS and then shaked for 10min. under 37 °C. The reagent so resulted is to be used for a series of tests described hereunder as the immobilized single stranded nucleic acid reagent (F).

And also, the immobilized single stranded nucleic acid reagent (B) under Example 7 was made in use here.

3) Preparation of labelled antibody

- under same procedure as was in the Example 7 -

4) Hybridization

PBS's of 500μl per each containing 0-, 5-, 10- and 20μl of reagent (E) preheated for 5 min. under 95°C then immediately cooled in ice, mixed with either (B) or (F) were brought to hybridization through shaking for 2hrs. under 60°C, and were washed twice using 0.01wt% Tween PBS (500μl).

5) Enzyme decomposition

- under same procedure as was in the Example 7 -

6) Detection

- under same procedure as was in the Example 7 -

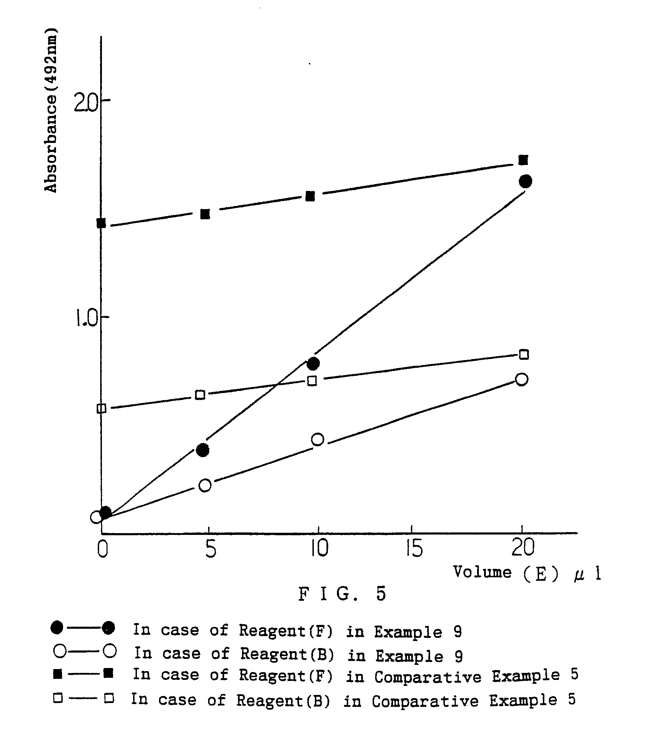

The results from the above detections of a nucleic acid are illustrated in Fig.5.

Comparative Example 5

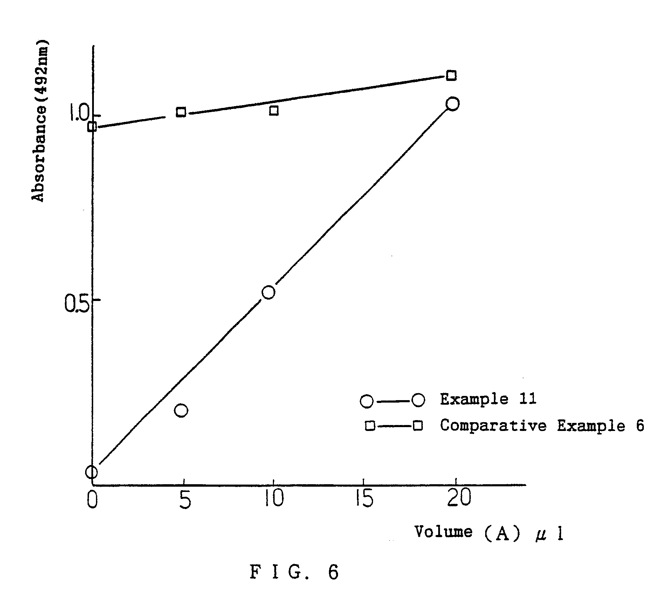

In case of Comparative Example 5, enzyme activity was determined in the same manner as in the Example 9 except that such nucleic acid which was not subjected to hybridization under the Example 9 was not digested using enzyme. The result obtained from the comparative Example 5 is illustrated in Fig. 6.

As a result it is evident that only such nucleic acid which acts complementarily with single stranded nucleic acid through enxyme digestion may be specifically, securely and rapidly detected.

Example 10

Those detective reagents under the present invention were prepared such that the following reagents were individually filled in a plug sealed flask.
1) Nucleic acid reagent (E) 2.4ml
2) Immobilized single straned nucleic acid reagent (F) 100units
3) Immobilized single straned nucleic acid reagent (B) 100units
4) Labelled antibody reagent (C) 24ml
5) Digestive enzyme reagent (D) 2.4ml
6) Buffer solution for use digestive enzyme reagent 120ml
7) PBS 240ml
8) 0.01wt% Tween-PBS 480ml
9) Physiological salt solution 2000ml
10) Substrate solution 120ml
11) Sulfric acid (1.5N) 700ml

These reagents were useful for the detection set forth in the Example 9. It is understood that the reagents 2), 3), 4)and 5) act as essential components for the present invention particularly in the above embodiment.

Example 11 (Detection of specific nucleic acid)

1) Proparation of single stranded nucleic acid complementarily acting within the specimen

- under same procedure as was in the Example 7 -

2) Preparation of immobilized single stranded nucleic acid

As the single stranded nucleic acid oligonucleotide having the same nucleotide sequence as is in the pBR322 Primer H available in marketplace and having its 5'- end (OH-residue) aminoalkylated using carbodiimidazol and hexamethylendiamine was obtained.

As the carrier ground glass beads of 6mmø with their surface amidated using γ-aminopropyl silane were made avialable.

The ground glass beads were intermixed with disuccinimidyl suberate (DSS) and the said aminoalkylated nucleotido to allow a reaction to proceed for 24hrs. under room temperature. The reactant so resulted had its concentration of nucleotide adjusted to 0.1 μg/ml and was subjected to the same treatment as was in the Example 7 to be made in use for a series of tests described hereunder as the immobilized single stranded nucleic acid reagent (G).

3) Preparation of anti nucleic acid monoclonal antibody

- under same procedure as was in the Example 7 -

4) Hybridization

PBS's of 500μl per each containing 0-, 5-, 10- and 20μl of reagent (A) preheated for 5 min. under 95 °C then immediately cooled in ice, mixed with (G) was brought to hybridization through shaking for 2hrs. under 60°C. The glass beads were washed twice using 0.01wt% Tween PBS (500 μl).

5) Enzyme decomposition

- under same procedure as was in the Example 7 -

6) Detection

- under same procedure as was in the Example 7 -

The results for the above detection of a nucleic acid are illutrated in Fig.6.

Comparative Example 6

In case of Comparative Example 6, enzyme activity was determined in the same manner as in the Example 11 except that such nucleic acid which was not subjected to hybridization under the Example 11 was not digested using enzyme. The result obtained from the comparative Example 6 is illustrated in Fig. 6.

It is evident that a constant molccule length maintained between the carrier and the single stranded nucleic acid advantageously serves for realizing a higher sensitivity and that only those nucleic acids which complementarily act with single stranded nucleic acid through enzyme treatment may be specifically, securely and rapidly detected.

Example 12

Those detective reagents under the present invention were prepared such that the following reagents were individually filled in a plug sealed flask.

1) Nucleic acid reagent (A) 1.2ml
2) Immobilized single straned nucleic acid reagent (G) 100units
3) Labelled antibody reagent (C) 12ml
4) Digestive enzyme reagent (D) 1.2ml
5) Buffer solution for use digestive enzyme reagent 60ml
6) PBS 120ml
7) 0.01wt% Tween-PBS 240ml
8) Physiological salt solution 1000ml
9) Substrate solution 60ml
10) Sulfric acid (1.5N) 350ml

These reagents were useful for the detection set forth in the Example 11. It is understood that the reagents 2), 3) and 4) act as essential components for the present invention particularly in the above embodiment.

**Claims**

1. A method for detecting a specific DNA through a labelled double stranded DNA comprising:
(a) attaching single stranded DNA fragments to a carrier,
(b) hybridizing at least a portion of the single stranded DNA fragments with complementary single stranded DNA fragments to form double stranded DNA, and
(c) attaching a labelled material with labelling compound to the double stranded DNA fragments.
2. Method in accordance with claim 1, wherein the carrier is at least one carrier selected from the group consist of siliceus carriers, magnetic carriers and organic carriers.

3. Method in accordance with claim 1, wherein the carrier is at least one material selected from the group consist of glass beads, nitrocellulose, polyvinyliden fluoride, Nylon and cellulose acetate.

4. Method in accordance with claim 1, wherein the labelling compound is at least one compound selected from the group consist of enzyme, chemical luminescent chromophores, biological luminescent chromopheres, fluoroscent chromophores, ligands with a specific binding capacity and isotopes.

5. Method in accordance with claim 1, wherein the labelled material is an antibody.

6. Method in accordance with claim 5, wherein the antibody is a monoclonal antibody.

7. Method in accordance with claim 1, wherein the respective process is carried out in one- or in several steps.

8. Reagents adapted for the method in accordance with claim 1 to be used for the said double stranded 'nucleic acid (DNA* -DNA hybrid) comprising single stranded DNA* and material with binding capacity label led with a labelling compound being reactive with double tranded nucleic acid (DNA* -DNA hybrid) obtained from hybridization with single stranded DNA*.

9. Reagents in accordance with claim 8, wherein the carrier is at least one compound selected from the group consist of glass beads, nitrocellulose, polyvinylidene fluoride, Nylon and cellulose acetate.

10. Reagents in accordance with claim 8, wherein the labelling compound is at least one compound selected from the group consist of enzyme, chemical luminescent chromophores, biological luminescent chromophores and fluorescent chromophores.

11. Reagents in accordance with claim 8, wherein the labelled material with binding capacity is an antibody.

12. A method for detecting a specific nucleic acid through a labelled double stranded nucleic acid comprising:
(a) hybridizing at least a portion of a single stranded nucleic acid fragments with complementary single stranded nucleic acid fragments to form double stranded nucleic acid,
(b) decomposing the single stranded nucleic acids fragments which are not hybridized, and
(c) attaching a label led material with labelling compound to the double stranded nucleic acid fragments.
13. Method in accordance with claim 12, wherein decomposing is carried out using an enzyme specifically digesting single stranded nucleic acids.
14. Method in accordance with claim 12, wherein single stranded nucleic acid is immobilized on a carrier.

15. Method in accordance with claim 14, wherein the carrier is at least one material selected from the group consist of siliceus carriers, magnetic carriers and organic carriers.

16. Method in accordance with claim 12, wherein the single stranded nucleic acid is of natural- or artificial origin.

17. Method in accordance with claim 16, wherein the single stranded nucleic acid is chemically synthesized.

18. Method in accordance with claim 12, wherein the labelled material is protein.

19. Method in accordance with claim 18, wherein the protein is an antibody.

20. Method in accordance with claim 12, wherein the label compound selected from the group consist of enzyme, chemical luminescent chromophres, biological luminescent chromophres, fluorescent chromophores, ligand with a specific binding capacity and isotopes.

21. Method in accordance with claim 12, wherein both the single stranded nucleic acid and the complementary single stranded nucleic acid are DNA.

22. Reagents adapted for the method under claim 12 to be used for detecting double stranded nucleic acid comprising: material(a) for decomposing a single stranded nucleic acid which was not hybridized and material (b) with binding capacity labelled by a labelling compound, which is capable to react with a nucleic acid which was made to double strands through hybridization.

23. Reagents in accordance with claim 22, wherein the material (a) is an enzyme which is capable to specifically digest a single stranded nucleic acid.

24. Reagents in accordance with claim 22, wherein the labelling compound is at least one compound selected from the group consist of enzyme, chemical luminescent chromophores, biological luminescent chromophores, fluorescent chromophores, ligands with specific bonding capacity and isotopes.

25. Reagents in accordance with claim 22, wherein the material with binding capacity is an antibody.

26. Reagents in accordance with claim 22, wherein the single stranded nucleic acid may be immobilized on at least a carrier selected from the group consist of siliceus carriers, magnetic carriers and organic carriers.

F I G. 1

F I G. 2

● ——● In case of Reagent(F) in Example 3
○ ——○ In case of Reagent(B) in Example 3
■ ——■ In case of Reagent(F) in Comparative Example 2
□ ——□ In case of Reagent(B) in Comparative Example 2

FIG. 3

F I G. 4

FIG. 5

● ——— ● In case of Reagent(F) in Example 9
○ ——— ○ In case of Reagent(B) in Example 9
■ ——— ■ In case of Reagent(F) in Comparative Example 5
□ ——— □ In case of Reagent(B) in Comparative Example 5

F I G. 6